# FASCICULE DE BREVET EUROPEEN

(11) **EP 0 503 312 B1**
(45) Date de publication et mention de la délivrance du brevet: **08.01.1997**
(21) Numéro de dépôt: 92102589.6
(22) Date de dépôt: 17.02.1992
(51) Int. Cl.: C07D 313/00, C11B 9/00

(54) **Procédé de préparation de la 15-pentadécanolide ou de mélanges riches en cette macrolide**
Verfahren zur Herstellung von 15-Pentadecanolid oder von an diesem Makrolid reichen Gemischen
Procedure for preparation of 15-pentadecanolide or mixtures rich in this macrolide

(30) Priorité: 13.03.1991 CH 766/91
(43) Date de publication de la demande: 16.09.1992
(73) Titulaire: FIRMENICH SA, CH-1211 Genève 8 (CH)
(72) Inventeur: Fankhauser, Peter, CH-1217 Meyrin (CH); Fantini, Piero, CH-1205 Genève (CH)
(74) Mandataire: Salvaterra-Garcia, Maria de Lurdes

(56) Documents cités:
- FR-A- 2 043 784

## Description

La présente invention a trait au domaine de la synthèse organique. Elle concerne, plus particulièrement, un procédé amélioré de préparation de la 15-pentadécanolide, ou de mélanges de celle-ci avec la 15-pentadéc-11(12)-énolide, par traitement thermique, dans un solvant organique, de bis-(13-oxabicydo[10.4.0]hexadéc-12-yl)péroxyde, le procédé étant caractérisé en ce que ledit solvant organique est choisi dans le groupe constitué par le m-diisopropylbenzène, le p-diisopropylbenzène, l'alcool benzylique, la N,N-diéthylamine, la benzylamine, la N-méthylbenzylamine, le phénylacétonitrile, la N-méthylpyrrolidone, la 2-pentylcydopentanone, le 1,2,3,4-tétrahydronaphtalène et les mélanges de ce dernier avec une amine.

La 15-pentadécanolide est une lactone fort appréciée en parfumerie pour son odeur musquée. Il est connu de l'art antérieur [voir, par exemple, le brevet FR 2043784 ou les brevets US 3,907,831 et 3,890,353] de préparer la 15-pentadécanolide et certains de ses dérivés au moyen d'une thermolyse d'un péroxyde de formule R'¹-O-O-R'² dans laquelle R'¹ représente un groupe de formule 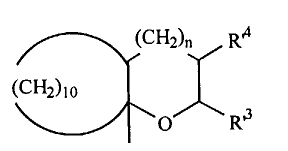 et R'² représente l'hydrogène, un hydrocarbure, un groupe acyle ou un deuxième groupe de la formule citée, dans laquelle R'³ et R'⁴ représentent l'hydrogène ou l'un d'eux un radical méthyle et l'autre l'hydrogène et n représente un nombre entier de valeur zéro à 3, la thermolyse s'effectuant dans un solvant organique, à une température comprise entre 80° et 150°C, suivie d'une hydrogénation du produit résultant. Parmi les péroxydes employés à titre de produits de départ dans ledit procédé figure précisément le bis-(13-oxabicydo[10.4.0]hexadéc-12-yl)péroxyde. Dans les documents cités de l'art antérieur, il est recommandé d'utiliser, en tant que milieu de thermolyse, des solvants dont le point d'ébullition se situe dans la gamme de températures comprises entre 80° et 150°C. Des hydrocarbures aromatiques liquides, tels le toluène, le xylène et des mélanges de o-, m- et p-xylène, sont cités à titre préférentiel et tous les exemples décrits font état de l'emploi du xylène en tant que solvant. Le produit de la réaction, après distillation, était représenté par un mélange plus ou moins riche en 15-pentadécanolide. Ce mélange était ensuite systématiquement hydrogéné pour fournir la 15-pentadécanolide qui était ainsi libérée de son dérivé insaturé, la 15-pentadéc-11(12)-énolide.

L'un des désavantages de ce procédé était constitué par le fait que le rendement de la réaction de thermolyse en ledit mélange de lactones, mesuré par rapport au péroxyde de départ, ne dépassait pas les 60%, voire était souvent inférieur à cette valeur. Un autre désavantage résidait dans le fait que la 15-pentadécanolide, obtenue après hydrogénation du mélange des lactones obtenues, était accompagnée de produits secondaires dont la séparation était laborieuse et coûteuse, ce qui entraînait des coûts supplémentaires et réduisait d'autant l'intérêt industriel du procédé.

Nous avons maintenant découvert, de façon inattendue, que ces désavantages peuvent être contrés par l'emploi de solvants particuliers lors du traitement thermique du péroxyde de départ, en particulier lorsque le bis-(13-oxabicydo[10.4.0]hexadéc-12-yl) péroxyde était employé en tant que produit de départ.

Les solvants dont il est question dans la présente invention sont des solvants organiques possédant une température d'ébullition comprise entre environ 170° et 250°C. La réaction est conduite par thermolyse à une température égale ou supérieure à environ 140°C, voire à la température d'ébullition ou à une température située au voisinage de la température d'ébullition du solvant choisi.

Parmi la variété de solvants choisis, le 1,2,3,4-tétrahydronaphtalène est employé de préférence, soit à l'état isolé, soit en mélange avec l'un des autres solvants susmentionnés, par exemple en mélange avec une base azotée telle la N,N-diéthylaniline.

La réaction de thermolyse est effectuée de la façon décrite dans les documents cités de l'art antérieur, inclus ici par référence. Selon un mode d'exécution particulier de l'invention, le procédé comprend en plus l'hydrogénation subséquente du mélange de thermolyse obtenu et cette hydrogénation est effectuée de façon usuelle. Les conditions spécifiques seront décrites dans le détail dans les exemples présentés plus loin.

Le bis-(13-oxabicyclo[10.4.0]hexadéc-12-yl)péroxyde, le produit de départ du procédé de l'invention, peut être préparé comme décrit dans l'art antérieur [voir les documents cités].

Comme il ressort des exemples présentés plus loin, le procédé de l'invention permet d'améliorer, de façon remarquable, le rendement en mélange de lactones obtenu après le traitement thermique du péroxyde (I). Par ailleurs, nous avons constaté avec surprise que, grâce au procédé de l'invention, l'on pouvait obtenir des mélanges dont la proportion en 15-pentadécanolide par rapport à son dérivé insaturé, 15-pentadéc-11(12)-énolide, était supérieure à celle des mélanges obtenus conformément au procédé de l'art antérieur. En effet, des mélanges ayant un contenu en 15-pentadéc-11(12)-énolide inférieur à 10% en poids, par rapport au poids du mélange, ont pu être obtenus. Ce résultat est fort intéressant car, pour certaines applications classiques de la 15-pentadécanolide en parfumerie, ces mélanges ont été jugés adéquats et peuvent remplacer celle-ci, ce qui rend superflue l'étape d'hydrogénation, auparavant indispensable pour obtenir une bonne qualité de 15-pentadécanolide. D'autre part, si l'on désire néanmoins effectuer l'hydrogénation sur le mélange obtenu par le procédé de l'invention, nous avons constaté que le rendement en 15-pentadécanolide est nettement meilleur et que la séparation de cette lactone par distillation est plus facile et économique.

Au vu de l'art antérieur cité, ces résultats sont tout à fait surprenants et inattendus. En effet, les solvants organiques dont il est question dans le procédé de l'invention présentent un point d'ébullition supérieur à la valeur recommandée dans les documents de l'état de la technique et l'homme du métier, s'inspirant de cet art antérieur, n'aurait pas eu une raison particulière d'y avoir recours, voire il y était découragé.

Comme indiqué plus haut, les mélanges de 15-pentadécanolide et 15-pentadéc-11(12)-énolide, contenant des quantités de cette dernière inférieures à 10% en poids du poids du mélange, obtenues par le procédé décrit, sont utiles à titre de produits intermédiaires pour la préparation de la 15-pentadécanolide. Elles sont également utiles à titre d'ingrédients parfumants en soi, étant à même de remplacer avec bonheur la 15-pentadécanolide dans des compositions parfumantes et produits parfumés.

L'invention sera décrite de façon plus détaillée à l'aide des exemples suivants dans lesquels les températures sont indiquées en degrés centigrades et les abréviations ont le sens usuel dans l'art.

### Exemple 1

La réaction est effectuée dans un réacteur de 21 à quatre cols, modifié par l'adjonction d'une sortie latérale permettant le soutirage en continu. Le réacteur était muni de :
- une canne d'introduction à double manteau, refroidi à l'eau,
- un condenseur à reflux, refroidi à l'eau,
- un agitateur mécanique,
- une sonde de température,
- une arrivée d'azote.
250 Ml de 1,2,3,4-tétrahydronaphtalène ont été chargés dans le réacteur maintenu sous atmosphère d'azote et, sous agitation vigoureuse, ils ont été chauffés à 140°. 77,2 G (0,127 mole) de bis-(13-oxabicyclo[10.4.0]hexadéc-12-yl)péroxyde en suspension dans 1200 ml de 1,2,3,4-tétrahydronaphtalène ont été ensuite ajoutés à l'aide d'une pompe doseuse au 1,2,3,4-tétrahydronaphtalène préchauffé tout en maintenant la température à environ 140°. Après environ 20 min, la solution réactionnelle a été recueillie par soutirage à travers la sortie latérale du réacteur, le débit de sortie étant réglé par celui de l'addition de la suspension de peroxyde. Une fois l'addition terminée, on a rincé la pompe doseuse à l'aide de 150 ml de 1,2,3,4-tétrahydronaphtalène, tandis que le contenu du réacteur a été maintenu à 140° pendant encore 10 min. Après refroidissement, on a réuni le contenu du réacteur à la solution réactionnelle soutirée.
Le mélange des lactones désirées a été enfin séparé par distillation fractionnée à l'aide d'un appareil muni d'une colonne Vigreux (rend. 73%).

### Exemple 2

En utilisant le même type de réacteur que celui décrit à l'exemple 1, on a procédé à la thermolyse de 85 g (0,178 mole) de bis-(13-oxabicyclo[10.4.0]hexadéc-12-yl)péroxyde en utilisant comme solvant du m-diisopropylbenzène (1200ml) au lieu du 1,2,3,4-tétrahydronaphtalène. Le rendement du mélange de lactones désirées était de 63%.
En opérant de manière analogue mais en utilisant les solvants indiqués ci-après on a obtenu les rendements décrits dans le tableau suivant.

**TABLEAU**

| N° essai | Solvant | Rendement [%] | P. Eb.¹⁾ [°C] |
|---|---|---|---|
| 1 | p-diisopropylbenzène | 59 | 210 |
| 2 | alcool benzylique | 69 | 205 |
| 3 | acétate de benzyle | 53 | 206 |
| 4 | acétophénone | 51 | 202 |
| 5 | N,N-diéthylaniline | 62 | 217 |
| 6 | benzylamine | 70 | 184 |
| 7 | N-méthylbenzylamine | 70 | 185 |
| 8 | N,N-diméthylbenzylamine | 61 | 180 |
| 9 | phénylacétonitrile | 70 | 232 |
| 10 | alcool furfurylique | 37 | 170 |
| 11 | succinate de méthyle | 58 | 192 |
| 12 | N-méthylpyrrolidone | 70 | 202 |
| 13 | acétoacétate de méthyle | 61 | 170 |
| 14 | 2-pentylcyclopentanone | 64 | 220 |
| 15 | triglyme ²⁾ | 51 | 225 |
| 16 | tétrahydronaphtalène + 2% N,N-diéthylaniline | 74 | 207(217) |

| | | | |
|---|---|---|---|
| 1) point d'ébullition du solvant choisi en °C à pression ordinaire | | | |
| 2) triéthylène glycol diméthyl éther. | | | |

## Revendications

1. Procédé pour la préparation de la 15-pentadécanolide, ou d'un mélange de 15-pentadécanolide et 15-pentadéc-11(12)-énolide, au moyen d'un traitement thermique, dans un solvant organique, de bis-(13-oxabicyclo[10.4.0]hexadéc-12-yl)péroxyde, le procédé étant caractérisé en ce que ledit solvant organique est choisi dans le groupe constitué par le m-diisopropylbenzène, le p-diisopropylbenzène, l'alcool benzylique, la N,N-diéthylaniline, la benzylamine, la N-méthylbenzylamine, le phénylacétonitrile, la N-méthylpyrrolidone, la 2-pentylcyclopentanone, le 1,2,3,4-tétrahydronaphtalène et les mélanges de ce dernier avec une amine.

2. Procédé selon la revendication 1, caractérisé en ce que le solvant est le 1,2,3,4-tétrahydronaphtalène ou un mélange de 1,2,3,4-tétrahydronaphtalène avec une amine.

3. Procédé selon la revendication 2, caractérisé en ce que ladite amine est la N,N-diéthylaniline.

4. Procédé selon l'une des revendications 1 à 3, caractérisé en ce que le produit de réaction obtenu après ledit traitement thermique est soumis à une hydrogénation subséquente.

## Patentansprüche

1. Verfahren zur Herstellung von 15-Pentadecanolid oder einer Mischung von 15-Pentadecanolid und 15-Pentadec-11(12)-enolid durch thermische Behandlung von Bis-(13-oxabicyclo[10.4.0]hexadec-12-yl)peroxid in einem organischen Lösungsmittel, dadurch gekennzeichnet, dass das oben genannte organische Lösungsmittel ausgewählt ist aus der Gruppe bestehend, aus m-Diisopropylbenzol, p-Diisopropylbenzol, Benzylalkohol, N,N-Diethylanilin, Benzylamin, N-Methylbenzylamin, Phenylacetonitril, N-Methylpyrrolidon, 2-Pentylcyclopentanon, 1,2,3,4-Tetrahydronaphthalin und Mischungen des letzteren mit einem Amin.

2. Verfahren gemäss Patentanspruch 1, dadurch gekennzeichnet, dass das Lösungsmittel das 1,2,3,4-Tetrahydronaphthalin oder eine Mischung des 1,2,3,4-Tetrahydronaphthalins mit einem Amin ist.

3. Verfahren gemäss Patentanspruch 2, dadurch gekennzeichnet, dass das oben genannte Amin N,N-Diethylanilin ist.

4. Verfahren gemäss einem der Patentansprüche 1 bis 3, dadurch gekennzeichnet, dass das nach der oben genannten thermischen Behandlung erhaltene Reaktionsprodukt einer nachfolgenden Hydrierung unterworfen wird.

## Claims

1. Process for the preparation of 15-pentadecanolide, or of a mixture of 15-pentadecanolide and 15-pentadéc-11(12)-enolide, by means of a thermal treatment, in an organic solvent, of bis-(13-oxabicyclo[10.4.0]hexadec-12-yl)peroxide, said process being characterized in that said organic solvent is selected from the group consisting of m-diisopropylbenzene, p-diisopropylbenzene, benzyl alcohol, N,N-diethylanyline, benzylamine, N-methylbenzylamine, phenylacetonitrile, N-methylpyrrolidone, 2-pentylcyclopentanone, 1,2,3,4-tetrahydronaphthalene and the mixtures of the latter with an amine.

2. Process according to claim 1, characterized in that the solvent is 1,2,3,4-tetrahydronaphthalene or a mixture of 1,2,3,4-tetrahydronaphthalene with an amine.

3. Process according to claim 2, characterized in that said amine is N,N-diethylanyline.

4. Process according to anyone of claims 1 to 3, characterized in that the reaction product obtained from said thermal treatment is subjected to subsequent hydrogenation.
